# EUROPEAN PATENT SPECIFICATION

(11) **EP 3 124 962 B1**
(45) Date of publication and mention of the grant of the patent: **28.09.2022**
(21) Application number: 15178758.7
(22) Date of filing: 29.07.2015
(51) Int. Cl.: G01N 27/12, G01N 33/00, B81B 7/02, H01L 23/31, B81C 1/00, H01L 23/00

(54) **GAS SENSOR ARRAY AND A METHOD FOR MANUFACTURING THEREOF**
GASSENSORARRAY UND EIN VERFAHREN ZUR HERSTELLUNG DESSELBEN
RÉSEAU DE CAPTEURS DE GAZ ET SON PROCÉDÉ DE FABRICATION

(43) Date of publication of application: 01.02.2017
(73) Proprietor: Sensirion AG, 8712 Stäfa (CH)
(72) Inventor: Steiner Vanha, Ralph, 8712 Staefa (CH); Mrcarica, Zeljko, 8712 Staefa (CH); Bürgi, Lukas, 8712 Staefa (CH); Bühler, Johannes, 8712 Staefa (CH); Steinmann, Lukas, 8712 Staefa (CH); Fuhrer, Samuel, 8712 Staefa (CH); Stämpfli, Marc, 8712 Staefa (CH); Wengle, Stefan, 8712 Staefa (CH); Schanz, Christoph, 8712 Staefa (CH)

(56) References cited:
- EP-A1- 2 765 410
- EP-A1- 2 952 886
- WO-A1-2010/140545
- DE-U1-202011 051 190
- DE-U1-202013 102 632

## Description

### Field of the Invention

The present invention relates to a gas sensor, a gas sensor array and a method for manufacturing thereof.

### Technical Background

An increasing number of gas sensors tend to be integrated into semiconductor chips.

Document EP 2 765 410 A1 discloses a gas sensor package comprising a gas sensor chip with a layer sensitive to a gas, and with a heater for heating the sensitive layer. Contact pads are provided for electrically contacting the gas sensor package and a die pad is provided for mounting the gas sensor chip to. Electrical connections connect the gas sensor chip and the contact pads. A molding compound at least partially encloses the gas sensor chip. An opening in the molding compound provides access to the sensitive layer of the gas sensor chip. One of the contact pads serves as a pin for supplying electrical current to the heater of the gas sensor chip.

A sensitive layer of such a gas sensor may be made from a material sensitive to one or more analytes. Such a material often needs to be annealed after being applied to the semiconductor chips. Conventional annealing steps, such as exposing the semiconductor chips to heat generated in an oven, might not be applicable to all gas sensors, e.g. they are not applicable if the gas sensor comprises parts, e.g. a package, which are sensitive to heat.

Therefore, a gas sensor and a method for manufacturing a gas sensor is desired that addresses these specifics.

### Disclosure of the Invention

The problem to be solved by the present invention is therefore, to provide a gas sensor and a method for the fabrication thereof, which allow to locally apply heat to a sensitive layer of a gas sensor chip.

This problem is solved by a method comprising the steps of claim 1, comprising the step of integrating a processing circuit in or with a gas sensor chip. Thereby, the processing circuit is preferably directly integrated in the gas sensor chip by CMOS fabrication techniques or is arranged together with the gas sensor chip on a substrate. The method further comprises the step of integrating a heater in the gas sensor chip. The gas sensor chip can comprise a semiconductor substrate and a layer stack arranged on the semiconductor substrate. The method further comprises a step of applying a sensitive material to the gas sensor chip, for building a layer sensitive to a gas. The sensitive material may be applied by contactless dispensing, for example by ink jet printing. The processing circuit can be configured for preprocessing signals from the sensitive material and for controlling the heater. A recess in the gas sensor chip may be manufactured underneath the heater by etching such as dry- or wet-etching, or otherwise removing material from the backside of the gas sensor chip, such as bulk substrate material. The remaining material of the gas sensor chip above the recess may form a membrane, which may include e.g. CMOS layers and/or parts of the bulk substrate material. Such a recess may serve for thermally isolating the membrane from the rest of the gas sensor chip, which preferably carries the processing circuit. Given that the sensitive layer will be applied to the membrane and the heater resides on or in the membrane, heat generated by the heater can only to a limited degree migrate into the other portions of the gas sensor chip.

A further step comprises the step of mounting the gas sensor chip onto a carrier, which carrier is a common carrier configured to accept multiple gas sensor chips. The common carrier can e.g. be designed as a ball grid array (BGA), or a an electrically conducting leadframe, which is understood as an electrically conducting structure of leads made out of metal, for example, by stamping or etching from a metal plate. When multiple gas sensors are to be fabricated on a single carrier, such as for an array of gas sensors, the carrier preferably comprises a die pad per gas sensor chip for mounting the gas sensor chip to, which die pad is understood as a flat portion of the carrier. An array of gas sensors comprises multiple gas sensor chips. The die pad preferably is made from an electrically and thermally conducting material, such as metal, as is the entire carrier. The gas sensor chip may be attached to the die pad in one example such that a substrate of the gas sensor chip may be grounded via the die pad. The die pad may have a footprint approximately equal to a footprint of the gas sensor chip. After having mounted the gas sensor chip to the die pad, the preferred recessed portion of the gas sensor chip and the die pad may form a cavity underneath the preferred membrane. Heat generated by the heater in the preferred membrane during operation may be transferred via gas in the cavity to the die pad acting as a heat sink. The carrier might further comprise a first subset of support leads, which are connected to the contact pads and a second subset of support leads, which are connected to the die pads. Multiple die pads may be provided per gas sensor in case other chips are to be arranged in the same gas sensor, such as an ASIC, etc. In another embodiment, the other chips are arranged on the same die pad as the gas sensor chip representing one gas sensor chip.

The carrier comprises sets of contact pads. Each set of contact pads is thereby electrically contacting one of the multiple gas sensor chips. One of the contact pads out of each set of contact pads of each gas sensor is a supply contact pad, for supplying electrical current to the processing circuit of the corresponding gas sensor chip. Rather than applying heat to each sensitive layer individually, it is envisaged to anneal the sensitive layer of the gas sensor chips when still residing on the common carrier. Therefore, a set of supply contact pads is electrically interconnected by electrical interconnects. Such set of supply contact pads can comprise two or more supply contact pads. The method comprises the step of applying current to at least one of the supply contact pads out of the set and via the interconnectors to all supply contact pads of the set, for supplying current from each of the supply contact pads to the corresponding processing circuits. In response to applying the current, a power controller, which is integrated in the corresponding gas sensor chip, is controlled by the corresponding processing circuit. In response to controlling the power controller, a supply current to the heater is enabled. The heater is thereby annealing the sensitive material, after the material is applied to the gas sensor chip. Via the interconnectors, the current is applied to all supply contact pads of the set and hence enabling a supply of current to the corresponding processing circuit. Preferably, at least one of the contact pads out of each set is a heater contact pad, connected to the heater. The heater is heated by applying a heater current to the heater contact pad, while controlling the corresponding power controller by the processing circuit. By interconnecting the supply contact pads of the set, current is supplied to each processing circuit and therefore the heater current to the corresponding heater contact pads can be controlled by the corresponding processing circuit via the power controller. The interconnection of the supply contact pads of the set therefore enables to simultaneously anneal the sensitive material of the corresponding gas sensor chips of the set.

In a preferred embodiment, the gas sensor comprises a one-time programmable (OTP) on-chip memory, for storing a heating routine. Therefore, in response to supplying the current to the processing circuit, the corresponding power controller is controlled according to the heating routine. Preferably, a flag stored in the one-time programmable on-chip memory indicates a state of the gas sensor chip, in particular a state of the sensitive material, e.g. whether it is annealed.

In a preferred embodiment, the heater contact pad and the supply contact pad serving as supply pin for supplying power for operating the gas sensor chip except for the heater are spaced apart from each other, e.g. are arranged at different edges of the back side of the gas sensor, and are electronically separated by the processing circuit. In this way, any heating of the gas sensor chip and its environment is introduced from different ends and does not accumulate in a hot spot as if the contact pads were neighbors.

In a preferred embodiment, the set of supply contact pads comprises supply contact pads of corresponding neighboring gas sensor chips. This means, that the gas sensor chips, which heaters are simultaneously supplied by current, are arranged neighborly on the carrier. Neighborly means, that they are arranged next to each other in any direction.

In a further preferred embodiment, the set of supply contact pads corresponds to gas sensor chips which are aligned in a row. This means, that the gas sensor chips, which heaters are supplied simultaneously by current, are arranged in a line. The advantage is thereby, that if all supply contact pads of the gas sensor chips of the carrier, which are aligned in a row, are interconnected by interconnectors, there is only one electrical probe required, to apply current to a first supply contact pad, for supplying current to all supply contact pads in the row simultaneously. Preferably, the interconnectors are fabricated by wire-bonding technique.

The sensitive layer may be made from a material being sensitive to one or more analytes. The sensitive layer may comprise multiple individual layer sections arranged next to each other and being separated from each for building a sensor array comprising a set of sensor cells, wherein a sensor cell may be understood as an entity of the gas sensor which may be read individually. Preferably, in the embodiment of the sensor array, each or at least some of the layer sections are suitable for sensing analytes, and in particular different analytes. Analytes may include one or more of, for example, H₂O, CO₂, NOX, ethanol, CO, ozone, ammonia, formaldehyde, or xylene without limitation. Specifically, the sensitive layer may contain a metal oxide material, and in particular a semiconducting metal oxide material, and specifically may contain metal oxide materials of different composition per layer section. A metal oxide material generally may include one or more of tin oxide, zinc oxide, titanium oxide, tungsten oxide, indium oxide and gallium oxide. Such metal oxides may be used for the detection of analytes such as VOCs, carbon monoxide, nitrogen dioxide, methane, ammonia or hydrogen sulphide. Metal oxide sensors are based on the concept that gaseous analytes interact with the metal oxide layer at elevated temperatures of the sensitive layer in the range of more than 100° Celsius, and specifically between 250°C and 350°Celsius. As a result of the catalytic reaction, the conductivity of the sensitive layer may change which change can be measured. Hence, such chemical sensors are also denoted as high temperature chemoresistors for the reason that a chemical property of the analyte is converted into an electrical resistance at high temperatures of the sensitive layer. Preferably, by means of such a gas sensor a gas may be investigated at least as to the absence or presence of the subject analyte/s the gas sensor is sensitive to. Hence, the gas supplied to the gas sensor may be analyzed by means of the sensitive layer as to if and which of the chemical substances or compounds the sensitive layer is sensitive to are present in the gas supplied. A combination of analytes detected in the gas supplied may suggest for a certain odour or for a certain gas. It is always subject to a design of the gas sensor as to how many different analytes and/or how many different properties of an analyte the gas sensor is sensitive to. In another embodiment, the sensitive material may comprise a polymer that in one embodiment may be sensitive to H₂O such that the sensor may be a humidity sensor. A capacity or a resistance of such polymer layer may be measured for deriving information as to the gas that may interact with the sensitive layer. The sensitive material can be applied before or after mounting the gas sensor chip onto the carrier.

In another embodiment of the present invention, a molding compound could be applied to each gas sensor chip of the carrier after the gas sensor chips are mounted to the common carrier and before or after the sensitive material is applied. The molding compound can at least partially enclose the gas sensor chip. A portion of the gas sensor chip can be left uncovered. An opening in the molding preferably provides access to at least the portion of uncovered area of the gas sensor chip. In case the sensitive material is applied after the molding compound, the sensitive material can then be applied through this opening onto the uncovered portion of the gas sensor chip. This order of manufacturing steps is in particularly preferred, if the molding process or other processing steps affect the sensitive material. The opening in the molding compound allows a gas to be measured to access the sensitive layer on the gas sensor chip collectively forming a gas sensor chip. At the same time, the opening allows to build the sensitive layer there through. It is not required that the entire portion uncovered by the molding compound is filled by the sensitive material. The molding compound is preferably not applied to the preferred membrane in order not to be in touch with this heated portion. The sensitive material may be applied to a section of this portion. The gas sensor chip may be bonded to the die pad, and such device may be molded with the molding compound not only serving as partial encapsulation of the gas sensor chip but also serving as mechanical fixture for the contact pads and the die pad.

The above specified manufacturing step of applying a mold to at least one of the gas sensor chips, also provides benefits in handling the device. In case the dispensing of the sensitive material may be executed by a different tool and/or at a different place than the mounting of the gas sensor chips on the carrier, the device preferably is transferred from one location to another one by a picker.

The molding compound which preferably is an epoxy with filler particles which filler particles e.g. may be glass, and specifically SiO₂, and which is applied to the gas sensor chip prior to applying the sensitive material is preferably configured to at least partially enclose and/or encapsulate the gas sensor chip. In case of the manufacturing of multiple gas sensors, the molding compound is applied to all gas sensor chips on the common carrier in a common manufacturing step thereby generating a continuous molding compound across the common carrier, including encapsulation of other chips if any.

For manufacturing the molding compound with the opening, a mold used in the molding process may have a protrusion. In this case, the gas sensor chip may be protected from mechanical impact during molding and the area for the designated sensitive layer may be sealed by a preferably elastic layer arranged on top of gas sensor chip in the area and/or arranged at the mold at the protrusion or at least parts thereof. After the molding, the layer may be removed again. In a different embodiment, a sealing frame may be deposited around an area the sensitive layer is expected to be arranged at. The protrusion of the mold for manufacturing the opening may then sit on the stress relief frame during molding. The sealing frame may not necessarily be removed after molding. The sealing frame may be made from elastic material. In another embodiment, a wall element may be arranged on top of the gas sensor chip encircling the area the sensitive layer is expected to be arranged at. Such wall element may, for example, either be bonded to the gas sensor chip or be manufactured by photolithographic steps, may be of a material different to the molding compound, and may act as a barrier preventing the molding compound from entering the area designated for the sensitive layer. In this embodiment, the mold may not necessarily need a protrusion but even can be flat and directly sit on the wall element during molding, e.g. on a sealing layer thereof. The wall element may not be removed after molding but continues to define the opening in the molding compound given that the molding compound stops at the wall. The wall preferably is of a material different than the molding compound.

It is preferred that the opening in the molding compound is arranged in a front side of the gas sensor while the contact pads and the die pad are arranged at a back side thereof. This provides a compact small size package suited for being arranged into portable electronic devices such as mobile phones, tablet computers, etc.

In a preferred embodiment, the gas sensor has a footprint with a length 1 times a width w in mm². In particular, the cuboid gas sensor has a footprint 1 x w mm² with 1 ∈ [2.3, 2.6] mm, w ∈ [2.3, 2.6] mm. It is preferred that the diameter d of the opening is less than 2 mm, and in particular d ∈ [1.4, 1.6] mm.

In a preferred embodiment, after mounting the or each gas sensor chip to the carrier comprising the contact pads and prior to applying the sensitive layer or the molding compound, the or each gas sensor chip is electrically connected to the assigned contact pads, either directly or via a separate ASIC, preferably by means of wire bonding.

In an alternate embodiment, the carrier may be a printed circuit board with the contact pads being formed by metallization on a front side of the printed circuit board, and the contact pads are formed by metallization on a backside of the printed circuit board which additionally requires vias through the printed circuit board for connecting the contact pads. Instead of a printed circuit board, another carrier such as a ceramic substrate or a glass substrate may be used.

In a first preferred severing step, the first subset of support leads, which are connected to contact pads, is severed before annealing the sensitive material and/or after applying the mold. Thereby, the contact pads can be isolated from the carrier. The gas sensor chip is still fixed to the carrier by the second subset of support leads. In another embodiment, the first subset of support leads can be insulating and thereby there is no need to sever it before annealing the sensitive material.

After annealing the sensitive material of the gas sensor chips, which are still mechanically coupled via the common carrier, they may be tested and/or calibrated simultaneously, e.g. by applying probes to the contact pads.

In a preferred second severing step, the second subset of support leads, the interconnectors, and the molding compound, if there is one, may subsequently be severed for separating the gas sensors. Severing comprises mechanically interrupting the support leads. The first and/or the second severing step preferably is applied to the subsets of support leads themselves from the back side of the present device, where typically portions of the carrier are accessible. Preferably, each die pad has a rectangular shape each with four edges, wherein the contact pads are arranged at opposite edges of the corresponding die pad. The support leads of the second subset, connecting each die pad, instead depart from the die pad at least from one of the other two edges. This enables severing the contact pads or respectively the first subset of support leads arranged at one edge of the corresponding die pad by a single severing step, such as a single sawing step. In the first severing step, it is preferred that the second set of support leads connecting the die pads is not severed yet. If a mold is applied to the gas sensor chip, the sensitive layers accessible through the openings are protected during dicing.

In one embodiment, a dicing tape can be attached to a front side of the present device thereby covering the openings in the molding compound. The device then is diced into individual gas sensor chips from the back side.

In a preferred embodiment, the locations to be severed may e.g. be marked, e.g. by etching.

After severing, the one or more severed interconnectors of an individual gas sensor are solely connected to the corresponding supply contact pad with one end. Preferably, the other end of each severed interconnector, which is not connected anymore and is insulated by an insulating layer or by fixing it to an insulating area on the chip.

In one embodiment, the gas sensor has six contact pads. As indicated above, a first one of the contact pads serves as pin for supplying a current to the processing circuit. Another one of the contact pads may serve as the heater supply pin for supplying power for operating the heater. A third one of the contact pads can serve as a ground pin. A fourth one of the contact pads can serve as a data pin for at least receiving measurement data from the gas sensor chip communicated according to a communication protocol. A fifth one of the contact pads serves as pin for a clock for operating the communication protocol, such as the I2C protocol. A sixth one of the contact pads serves as a programmable pin for programming the gas sensor chip.

Preferably, the six contact pads are arranged in two rows by three at the backside of the gas sensor and the die pad is arranged between the two rows of three contact pads each. This allows for a dense arrangement, in particular if the die pad is of rectangular shape.

In a preferred embodiment, the front side of the gas sensor comprises at least one marking. In case the footprint of the gas sensor is of rectangular shape each marking is arranged in a corner of the front side. Preferably, a single marking is applied to one of the corners for defining an orientation of the gas sensor.

The present gas sensor in its various embodiments not only is small in its dimensions but at the same time reduces outgassing possibly impacting measurements, ensures a stable / "clean" power supply V_{DD} while at the same time supplying a high current to the heater by the heater contact pad not interfering with the power supply to all functions of the gas sensor except the heater. The heating of the sensitive layer is only locally applied, where temperatures of more than 200 ° degrees Celsius and sometimes even far more may be achieved. In contrast, outside the preferred membrane with the heater and the sensitive layer the temperature may not exceed e.g. 85 ° degrees Celsius for avoiding an impact on processing sensor signals. A corresponding processing circuit preferably is arranged outside the membrane and preferably supplies digital signals to one of the contact pads. The present gas sensor preferably finally can be SMD mounted to an external support.

Other advantageous embodiments of the manufacturing of a gas sensor are listed in the dependent claims as well as in the description below.

### Brief Description of the Drawings

Embodiments of the present invention, aspects and advantages will become apparent from the following detailed description thereof. Such description makes reference to the annexed drawings, wherein the figures show:
Fig. 1 in diagrams a) to c) manufacturing steps as applied in an embodiment of a method according to the present invention,
Fig. 2 in diagrams a) to c) manufacturing steps as applied in an embodiment of a method according to the present invention,
Fig. 3 in diagrams a) to b) manufacturing steps for a molding compound as applied in an embodiment of a method according to the present inventions,

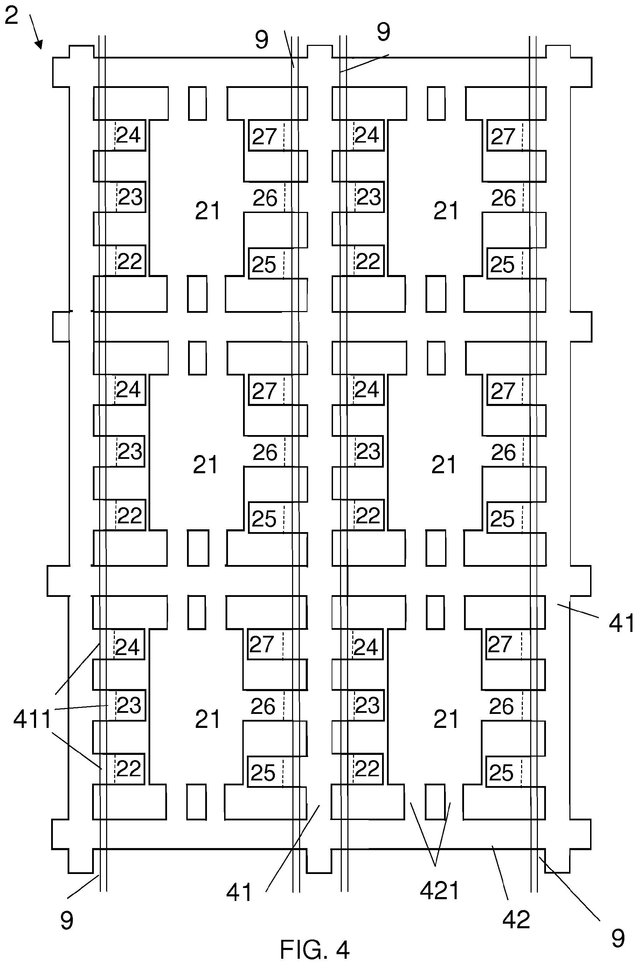

Fig. 4 a further manufacturing step as applied in an embodiment of a method according to the present invention, and
Fig. 5 a gas sensor according to an embodiment of the present invention, in a perspective view in diagram a), in a top view in diagram b), in a bottom view in diagram c), and in a cut in diagram d).

### Detailed Description of the Drawings

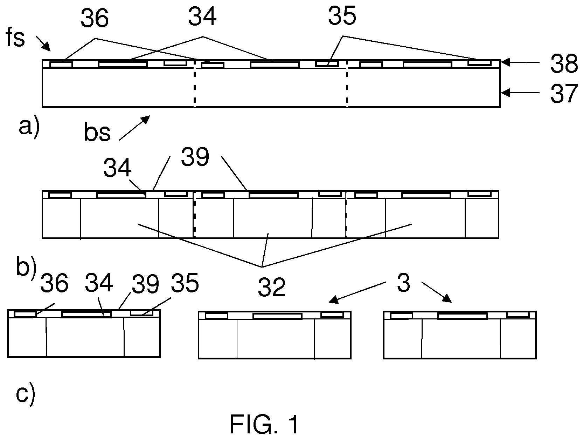

Figure 1 illustrates in diagrams a) to c) manufacturing steps as applied in a method for manufacturing a gas sensor according to an embodiment of the present invention. These steps refer to the manufacturing of suitable gas sensor chips that later will be packaged into gas sensors. In diagram 1 a), a wafer is provided containing a semiconductor substrate 37 and a layer stack 38 arranged on the semiconductor substrate 37 at a front side fs of the wafer. The wafer as such may be a standard CMOS wafer into which integrated processing circuits 36 are manufactured, as well as contact pads 35, for example. The vertical dashed lines in the wafer indicate dicing trenches along which the wafer will be diced into multiple gas sensor chips later on. In addition to bond pads 35 and processing circuits 36, heaters 34 are manufactured in the front side fs of the wafer, e.g. embodied in one of the patterned metal layers of the layer stack 38.

After having prepared the wafer according to diagram 1 a), preferably recesses 32 are manufactured into a backside bs of the wafer, and specifically underneath each heater 34. The recesses 32 may in one embodiment be manufactured by etching into the semiconductor substrate 37. By doing so, each future gas sensor chip may comprises a membrane 39 that may be built from the layer stack 38 and possibly from a thin portion of the semiconductor substrate 37. The preferred membrane 39 may serve for thermal insulation.

According to diagram 1 c), the wafer then may be diced into individual gas sensor chips 3, each gas sensor chip 3 may comprise a membrane 39 at its front side fs, and comprises a heater 34 deposited on or integrated into the preferred membrane 39, a processing circuit 36 and bond pads 35 for electrically contacting the gas sensor chip 3, both preferably arranged outside the preferred membrane 39.

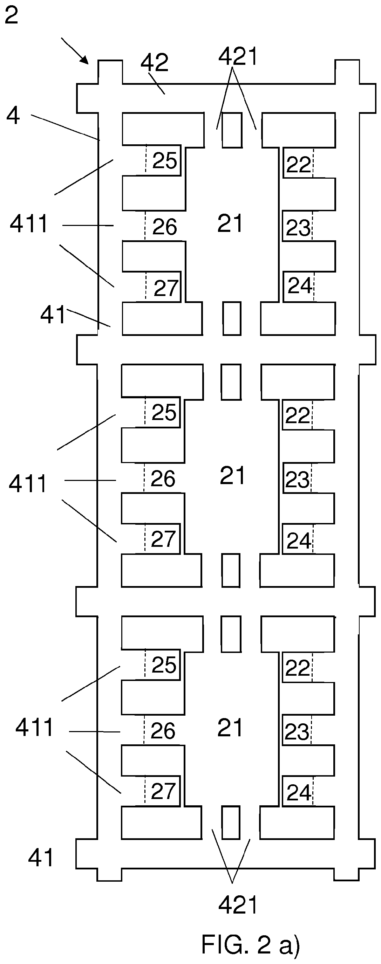

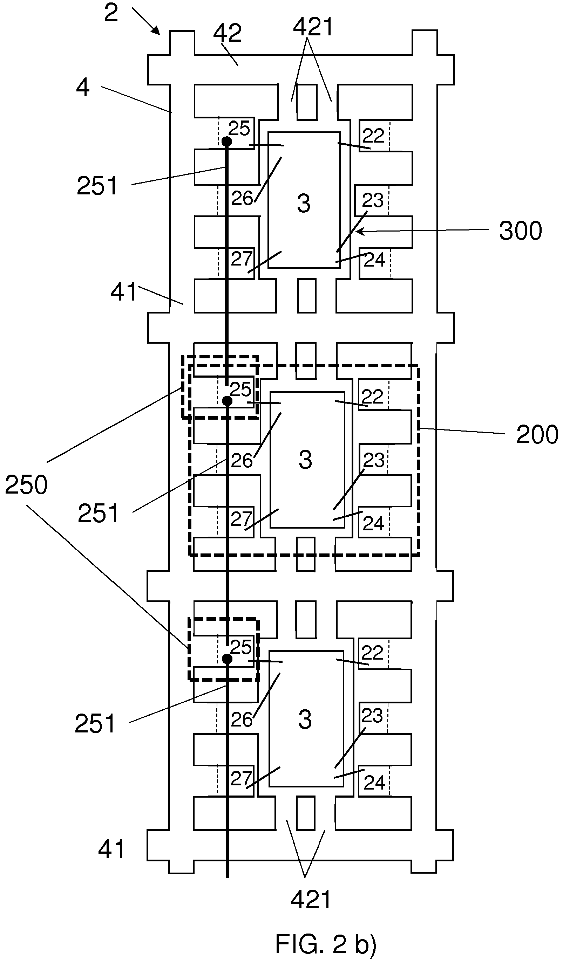

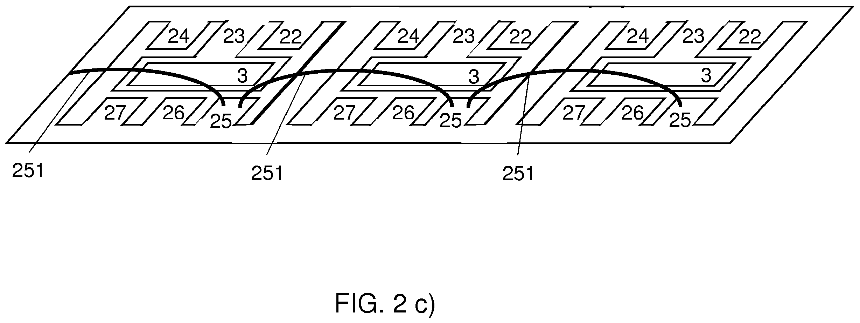

Figure 2 illustrates in diagrams a) to c) manufacturing steps as applied in a method according to an embodiment of the present invention.

According to diagram 2 a), a cutout of a carrier 2, which has preferably the form of a leadframe 4, is provided, which is shown in diagram 2 a). A leadframe 4 typically is a grid like structure made from an electrically conducting material. The individual interconnected leads may be etched or stamped from a thin plate of metal. It is assumed, that the leadframe 4 already is prepared to a shape as shown in diagram 2 a) for the purpose of manufacturing an array of gas sensors thereon. Although in the present example, the leadframe 4 comprises only three platforms for building an array of gas sensors, it is understood, that in real manufacturing the leadframe 4 may contain many more platforms, e.g. for building tens or hundreds of sensors on. Hence, the leadframe 4 contributes to a carrier 2 that is common to all the gas sensors which are desired to be manufactured in a common manufacturing process.

The present leadframe 4 contains, for example, vertical support leads 41 and horizontal support leads 42. The vertical support leads 41 contain finger like extensions, the end sections of which extensions represent contact pads 22-27 of the future gas sensors. Those end sections are indicated by dashed lines, while the extensions as such are referred to as a first subset of contact support leads 41, 411. The leadframe 4 further comprises die pads 21, each die pad 21 serving as a platform to mount a gas sensor chip 3 on. A second subset of support leads 42, 421 is connected to the die pad 21.

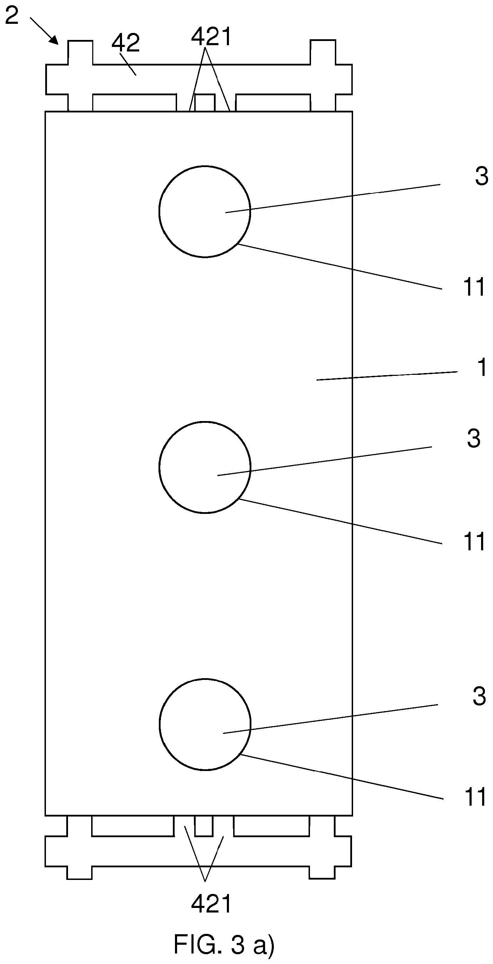

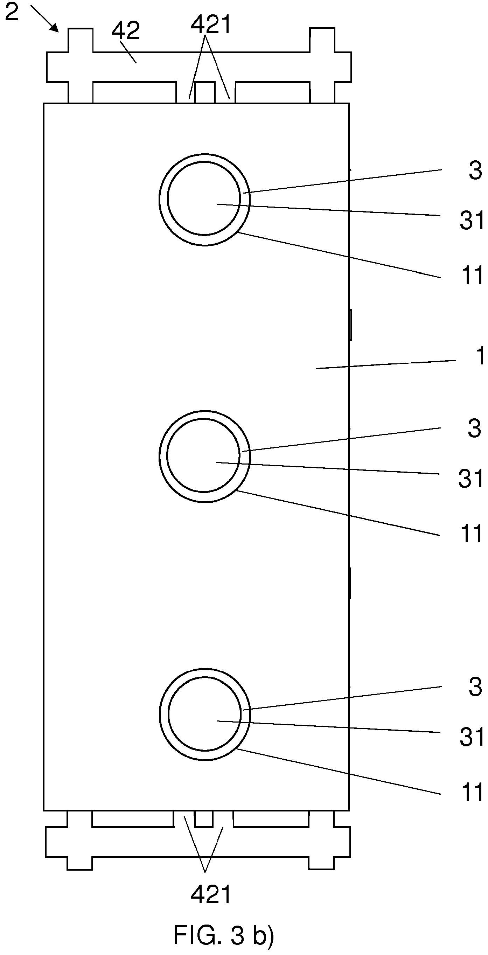

In a next step, as is shown in diagram 2b), a gas sensor chip 3 is mounted on each die pad 21. The gas sensor chips 3 may be the gas sensor chips 3 prepared according to Figure 1. A sensitive material is applied onto the front side of the gas sensor chip 3. The material can be applied before or after mounting the gas sensor chip 3 onto the carrier. In case a molding compound is applied to the gas sensor chip 3, the sensitive material can even be applied after the molding compound is applied as shown in Fig. 3 b). Each gas sensor chip 3 may be placed on the corresponding die pad 21 with its back side bs facing the die pad 21, such that a cavity is built from the recessed portion of the gas sensor chip 3 and the die pad 21. In a next step, the gas sensor chip 3 is electrically connected to the contact pads 22-27 by wire bonding as is indicated by bond wires 300. The contact pads 22-27 which contact the same gas sensor chip 3 form a set of contact pads 200. One of the contact pads 22-27 out of each set of contact pads 200 is a supply contact pad 25. The supply contact pad 25 supplies electrical current to the processing circuit 36 of the corresponding gas sensor chip 3.. Two or more supply contact pads 25 build a set of supply contact pads 250. The supply contact pads 25 of the set 250 are interconnected by electrical interconnectors 251. For annealing a sensitive layer of the corresponding gas sensor chip 3, a current is applied to at least one supply contact pad 25 out of the set of supply contact pads 250, thereby applying current to all supply contact pads 25 of the set 250 via the interconnectors 251. Fig. 2c) shows an oblique view of the gas sensor array surface of Fig. 2b). The interconnectors 251 interconnect the supply contact pads 25 of a set 250, wherein the interconnectors 251 are preferably wire-bonds. One or more of such wire-bonds can contact one of the supply contact pads 25. Preferably, as shown in the Figure 2c), some supply contact pads 25 are connected with two wire-bonds to the supply contact pads 25 of the corresponding neighboring gas sensor chips 3 out of the set of supply contact pads 250.

In a preferred step of the method shown in Figure 3a, a molding compound 1 is applied on top of the present structures such that the molding compound 1 encapsulates the corresponding gas sensor chips 3 except for an opening 11 which opening 11 provides access to a portion of each gas sensor chip 3 which preferably is the membrane 39. The molding compound 1 provides mechanical stability and protects the gas sensor chip 3. The molding compound 1 preferably is made from insulating material, such as epoxy containing filler particles such as glass, specifically SiO₂. For manufacturing the molding compound 1 the device of diagram 3 a) is inserted into a mold. The mold is then filled, e.g. by a liquid molding compound. Thereafter, the molding compound 1 may harden into a solid molding compound 1 such as referred to in diagram 3 a). As can be seen from this diagram, the molding compound extends over the carrier 2 into a continuous molding compound except for the openings 11.

If a molding compound 1 is applied to the gas sensor chip 3 according to Figure 3 a), it is shown in Figure 3 b) how the sensitive material is applied through each opening 11 and onto the respective portions of the gas sensor chips 3 for forming a sensitive layer 31. For the manufacturing of the gas sensor, with or without the molding compound 1, the sensitive material may be contactless dispensed onto the gas sensor chip 3, e.g. by means of the print head of an ink jet printer that supplies sensitive material in form of a solution or suspension.

Preferably, after applying the sensitive material to the gas sensor chip 3, before or after applying the molding compound 1, or without a molding compound 1, a first severing step is performed. In this first severing step, it is preferred to sever a first subset of support leads from the back side of the device which present back side is shown in diagram 4. The result can be seen in the bottom view according to diagram 4. Sawing lines 9 are indicated along which the first subset of support leads 41, 411 to the contact pads 22-28 of the gas sensor array will be severed. Instead of two slim sawing lines 9 as shown in diagram 4, a wide sawing line can be provided for severing the contact pads 22-27 assigned to two different die pads 21 in a single sawing step. A second set of support leads 42, 421 connecting the die pads 21 remains intact, i.e. it is only vertically sawed.

After the first severing step, current may be applied to the interconnectors 251, the supply contact pads 25 and the second subset of support leads 42 which connect via the die pads 21 to the contact pads 23 serving as ground pins. Thereby, current is supplied to the corresponding processing circuits 36. In response to applying the current, corresponding power controllers, which are integrated in the corresponding gas sensor chips 3, are controlled by the corresponding processing circuits 36. The power controllers enable supply of current to the corresponding heaters 34, for annealing the corresponding sensitive layers 31. The layer 31 of sensitive material can then be annealed simultaneously on all gas sensor chips 3, while still residing on the common carrier 2, by applying current, e.g. by only one probe, to one supply contact pad 25 out of the set 250.

In further steps, the gas sensors on the common carrier 2 may be calibrated and/or tested, e.g. by applying electrodes to the contact pads 22-27 from the back side.

In a final step, the gas sensors may be separated from each other by a second severing step, wherein the second subset of support leads 42, 421, the electrical interconnector 251 and the molding compound 1, are preferably severed from the back side. Thereby, a front side of the gas sensor can be protected by attaching it to a dicing tape.

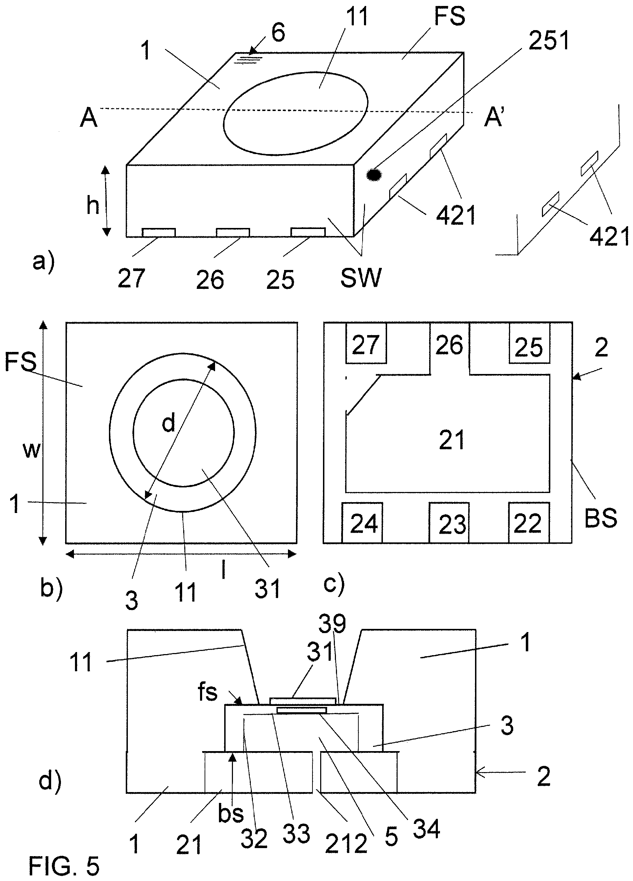

Figure 5 illustrates an individual gas sensor comprising a molding compound 1 according to an embodiment of the present invention which preferably is manufactured according to an embodiment of a manufacturing method, and specifically according to the manufacturing method illustrated in Figure 2 and 3. Diagram 5 a) illustrates an embodiment of the gas sensor with a molding compound in a perspective view, diagram 5 b) in a top view, diagram 5 c) in a bottom view, and diagram 5 d) in a cut view along lines A-A' of diagram 5 a).

The gas sensor with the molding compound 1 has the shape of a cuboid which is defined by a molding compound 1 and which has a front side FS and a back side BS opposite to the front side FS. An opening 11 in the molding compound 1 provides access to a sensitive layer 31 of a gas sensor chip 3. In one of the corners of the molding compound 1 on the front side FS, a marking 6 is provided, e.g. by laser processing, ink jet printing, etc. The marking 6 preferably serves for indicating an orientation of the gas sensor, but may instead or additionally also indicate one or more of a device number, a serial number, the manufacturer, etc. In side walls SW, front ends of contact pads 25, 26, 27 are exposed from the molding compound 1 as well as front ends of support leads 421. The electrical interconnector 251 may be partly enclosed by the mold and severed together with the mold, such that only one end is visible as shown in Figure 5 a).

However, in a different embodiment, one or more of the contact pads and/or the die pad supports 211 may not finalize with the bottom edge of the gas sensor as shown in Figure 1, but may be elevated from such edge such as shown in the cutout to the right of Figure 1, where the die pad supports 211 are arranged at a distance of e.g. between 100 µm and 200 µm from the bottom edge.

The gas sensor with the molding compound 1 of the present example has a height h of preferably between 0.7 and 0.8 mm. The opening 11 is of circular shape and has a diameter d, less than 2 mm, and preferably between 1.4 and 1.6 mm. The gas sensor has a footprint 1 × w with each of the length 1 and the width preferably being in the range between 2.3 and 2.7 mm. The sensitive layer 31 preferably has a diameter little smaller than the diameter d of the opening such that a small portion of the gas sensor chip 3 outside the sensitive layer 31 is visible in the top view.

The back side BS of the gas sensor, see diagram 5 c), shows six contact pads 22-27. The contact pads 22-27 are arranged in two rows of three contact pads each at two opposite edges of the gas sensor. Preferably, each contact pad is dimensioned by less than 0.5 mm by 0.5 mm, and a pitch between each two contact pins of a row is in the range of 0.8 mm. Preferably, the contact pad 25 serves as supply pin for supplying electrical current to processing circuit 36. Preferably, one of the other contact pads serves as a heater pin for supplying current to the heater 34, if the power controller is enabled. Another contact pad can serve as a ground pin. A further contact pad can serve as a data pin for at least receiving measurement data from the gas sensor chip 3 communicated according to a communication protocol, such as the I2C protocol. A fourth contact pad can serve as pin for a clock for operating the communication protocol, such as the I2C protocol. A contact pad can also serve as a programmable pin for programming the gas sensor chip 3, e.g. with calibration data. In between the two rows, a die pad 21 is provided which serves as support for the gas sensor chip 3. The die pad 21 is of rectangular shape and has one flattened corner which may serve as an optical and/or mechanical encoding for an orientation of the gas sensor. The contact pads 22-27 and the die pad 21 are mechanically linked by the molding compound 1.

According to the cut view according to diagram 5d), the gas sensor chip 3 is arranged on top of the die pad 21, and e.g. is bonded thereto. The gas sensor chip 3 has a front side fs and a back side bs. In one embodiment, the gas sensor chip 3 may comprise a semiconductor substrate 37 and CMOS layer arranged on top of the substrate 37. The substrate 37 may be etched or otherwise partially removed from the backside bs such that the gas sensor sensor chip 3 may have a recess 32 on its backside bs. As a result of building the preferred recess 32 in the gas sensor chip 3, a thinned structure is generated in the gas sensor chip 3, also referred to as membrane 39. The sensitive structure 31 is arranged on or in the preferred membrane 39.

In a specific embodiment, the sensitive layer 31 comprises a metal oxide layer which is to be heated for enabling the sensing of chemical analytes. For this purpose, a heater 34 such as a resistive heater is arranged in or under the membrane 33 for heating the sensitive layer 31. Hence, both the gas sensitive layer 31 and the heater 34 may be arranged on or in the membrane 39 above the recess 32. This arrangement is owed to a thermal insulation the preferred membrane 39 provides which improves the accuracy of the measurement.

In a preferred embodiment, the die pad 21 has a hole 212 for connecting the cavity 5 to the outside world. Heat may be transferred via gas in the cavity 5 to the die pad 21 which acts as a heat sink. The hole 212 in the die pad may allow balancing of pressure which may be beneficial in view of the heating of the heater.

While above there are shown and described embodiments of the invention, it is to be understood that the invention is not limited thereto but may be otherwise variously embodied and practised within the scope of the following claims.

## Claims

1. Method for manufacturing a gas sensor, comprising the steps of
- integrating a processing circuit (36) in a gas sensor chip (3) or arranging a processing circuit (36) together with a gas sensor chip (3), on a substrate (37),
- integrating a heater (34) in the gas sensor chip (3),
- applying a sensitive material to the gas sensor chip, for building a layer sensitive to gas,
- mounting multiple gas sensor chips (3) onto a carrier (2), which carrier (2) is a common carrier (2) configured to accept the multiple gas sensor chips (3), wherein the common carrier (2) comprises sets (200) of contact pads (22-27), wherein each set of contact pads (200) electrically contacts one of the multiple gas sensor chips (3), wherein one of the contact pads (22-27) out of each set (200) is a supply contact pad (25), for supplying electrical current to the processing circuit (36) of the corresponding gas sensor chip (3),
- electrically interconnecting a set of supply contact pads (250) by electrical interconnectors (251),
- applying a current to at least one of the supply contact pads (25) out of the set (250) and via the interconnectors (251) to all supply contact pads (25) of the set (250), for supplying current to the corresponding processing circuits (36),
- in response to applying the current, controlling a power controller, which is integrated in the corresponding gas sensor chip, by the corresponding processing circuit (36), and
- in response to controlling the power controller, enabling a supply of current to the heater (34), for annealing the corresponding sensitive layer (31).

2. Method according to claim 1,
wherein the set of supply contact pads (250) comprises supply contact pads (25) of corresponding neighboring gas sensor chips (3).

3. Method according to any one of the previous claims,
wherein the set of supply contact pads (250) corresponds to gas sensor chips (3) which are aligned in a row,
preferably wherein the electrical interconnectors (251) are fabricated by wire-bonding.

4. Method according to any one of the previous claims, comprising
- applying for each gas sensor chip (3) a molding compound (1) at least partially enclosing the gas sensor chip (3) thereby generating an opening (11) providing access to a portion of the gas sensor chip (3) being uncovered by the molding compound (1),
- applying the sensitive material for each gas sensor chip (3) after the molding compound (1) is applied,
- applying the sensitive material through the opening (11) onto the uncovered portion of the gas sensor chip (3).

5. Method according to any one of the previous claims,
wherein the carrier (2) comprises die pads (21) for mounting the gas sensor chips (3) to, a first subset of support leads (41, 411) connected to the contact pads (22-27) and a second subset of support leads (42, 421) connected to the die pads (21),
the method comprising
- a first severing step, severing the first subset of support leads (41, 411) before annealing the sensitive layer (31),
- a second severing step, severing the electrical interconnectors (251) and the second subset of support leads (42, 421), after annealing the sensitive layers (31), for separating the gas sensor chips (3),
in particular wherein the first and/or the second severing step is done by sawing.

6. Method according to claim 5,
wherein the first severing step is applied after the sensitive material is applied and/or after the molding compound (1) is applied.

7. Method according to claim 5,
wherein the gas sensor chips (3) are tested and/or calibrated after having annealed the sensitive layers (31) and prior to the second severing step.

8. Method according to claim 4,
wherein the sensitive material is applied to the uncovered portion of the gas sensor chip (3) by contactless dispensing, and in particular by ink jet printing.

9. Method according to any one of the previous claims,
wherein at least one of the contact pads (22-27) out of each set (200) is a heater contact pad (23), connected to the heater (34),
- applying a heater current to the heater contact pad (23) while the corresponding power controller is controlled, thereby heating the heater (34).

10. Method according to any one of the previous claims,
wherein a one-time programmable on-chip memory is arranged in the gas sensor, for storing a heating routine, and
wherein in response to supplying the current to the processing circuit (36), the corresponding power controller is controlled according to the heating routine.

11. Method according to claim 10,
- indicating a state of the gas sensor chip (3) by a flag in the one-time programmable on-chip memory,
- controlling the power controller dependent on the flag, and
- in particular, controlling the power controller only when the flag indicates a non-annealed state of the sensitive material.

12. Gas sensor array comprising
- multiple processing circuits (36), and
- multiple gas sensor chips (3), each of the gas sensor chips (3) comprising:
- a heater (34),
- a processing circuit (36) integrated in or with the gas sensor chip (3),
- a sensitive material, applied to the gas sensor chip (3) for building a layer (31) sensitive to a gas,
- a power controller, integrated in the gas sensor chip (3), which is controlled by the processing circuit (36),
wherein for each gas sensor chip (3), the corresponding processing circuit (36) is integrated in the gas sensor chip (3) or the processing circuit (36) is arranged together with the gas sensor chip (3) on a substrate,
the array further comprising
- a carrier (2), which carrier (2) is a common carrier (2) with multiple gas sensor chips (3) being mounted to, wherein the common carrier (2) sets of contact pads (200), wherein each set of contact pads (200) electrically contacts one of the multiple gas sensor chips (3), wherein one of the contact pads (25) out of the set (200) is a supply contact pad (25), for supplying electrical current to the processing circuit (36) of the corresponding gas sensor chip,
- electrical interconnectors (251) for electrically interconnecting a set of supply contact pads (250), for enabling the supply of a current to all supply contact pads (25) of the set (250) and hence to the corresponding processing circuits (36).

13. Gas sensor array according to claim 12,
wherein the set of supply contact pads (250) corresponds to neighboring gas sensor chips (3),
wherein the electrical interconnectors (251) are preferably wire-bonds.

## Patentansprüche

1. Verfahren zur Herstellung eines Gassensors,
umfassend die Schritte:
- Integrieren einer Verarbeitungsschaltung (36) in einem Gassensorchip (3) oder Anordnen einer Verarbeitungsschaltung (36) zusammen mit einem Gassensorchip (3) auf einem Substrat (37),
- Integrieren eines Heizers (34) in den Gassensorchip (3),
- Aufbringen eines empfindlichen Materials auf den Gassensorchip zum Aufbau einer gassensitiven Schicht,
- Aufbringen mehrerer Gassensorchips (3) auf einen Träger (2),
wobei der Träger (2) ein gemeinsamer Träger ist, (2) konfiguriert, um die mehreren Gassensorchips (3) aufzunehmen,
wobei der gemeinsame Träger (2) Sätze (200) von Kontaktpads (22-27) umfasst, wobei jeder Satz von Kontaktpads (200) einen der mehreren Gassensoren (3) elektrisch kontaktiert,
wobei eines der Kontaktpads (22-27) aus jedem Satz (200) ein Versorgungskontaktpad (25) ist zum Zuführen von elektrischem Strom zu der Verarbeitungsschaltung (36) des entsprechenden Gassensorchips (3),
- elektrisches Verbinden eines Satzes von
Versorgungskontaktpads (250) durch elektrische Zwischenverbindungen (251),
- Anlegen eines Stroms an mindestens eines der Kontaktpads (25) aus dem Satz (250) und über die Zwischenverbindungen (251) zu allen Versorgungskontaktpads (25) aus dem Satz (250), um die entsprechende Verarbeitungsschaltung (36) mit Strom zu versorgen,
- als Reaktion auf das Anlegen des Stroms durch die entsprechenden Verarbeitungsschaltung (36) einen Leistungsregler Steuern, der in den entsprechenden Gassensorchip integriert ist, und
- als Reaktion auf die Steuerung des Leistungsreglers eine Stromversorgung des Heizers (34) Ermöglichen zum Ausheilen der entsprechenden sensitiven Schicht (31).

2. Verfahren nach Anspruch 1,
wobei der Satz von Versorgungskontaktpads (250) Versorgungskontaktpads (25) von entsprechenden benachbarten Gassensorchips (3) umfasst.

3. Verfahren nach einem der vorhergehenden Ansprüche,
wobei der Satz von Versorgungskontaktpads (250) Gassensorchips (3) entspricht, die in einer Reihe ausgerichtet sind,
vorzugsweise wobei die elektrischen Zwischenverbinder (251) durch Drahtbonden hergestellt werden.

4. Verfahren nach einem der vorhergehenden Ansprüche, umfassend
- Aufbringen einer Vergussmasse (1) für jeden Gassensorchip (3), die den Gassensorchip (3) zumindest teilweise umschließt, wodurch eine Öffnung (11) erzeugt wird, die einen Zugang zu einem Teil des Gassensorchips (3), der nicht von der Vergussmasse (1) bedeckt ist, ermöglicht,
- Aufbringen des sensitiven Materials für jeden Gassensorchip (3) nach dem Aufbringen der Vergussmasse (1),
- Aufbringen des sensitiven Materials durch die Öffnung (11) auf den unbedeckten Teil des Gassensorchips (3).

5. Verfahren nach einem der vorhergehenden Ansprüche,
wobei der Träger (2) Chippads (21) zum Anbringen der Gassensorchips (3) daran, eine erste Teilmenge von Hilfsleitungen (41, 411), die mit den Kontaktflächen (22-27) verbunden sind, und eine zweite Teilmenge von Hilfsleitungen (42, 421), die mit den Chippads (21) verbunden sind, umfasst, wobei das Verfahren umfasst
einen ersten Trennschritt, der die erste Teilmenge von Hilfsleitungen (41, 411) vor dem Ausheilen der empfindlichen Schicht (31) trennt,
- einen zweiten Trennschritt, der die elektrischen Verbindungsleitungen (251) und die zweite Teilmenge von Hilfsleitungen (42, 421) nach dem Ausheilen der sensitiven Schichten (31) trennt, zum Vereinzeln der Gassensorchips (3), insbesondere wobei der erste und/oder der zweite Trennschritt durch Sägen erfolgt.

6. Verfahren nach Anspruch 5,
wobei der erste Trennschritt nach dem Auftragen des sensitiven Materials und/oder nach dem Auftragen der Vergussmasse (1) erfolgt.

7. Verfahren nach Anspruch 5,
wobei die Gassensorchips (3) nach dem Ausheilen der sensitiven Schichten (31) und vor dem zweiten Trennschritt getestet und/oder kalibriert werden.

8. Verfahren nach Anspruch 4,
wobei das sensitive Material auf den unbedeckten Teil des Gassensorchips (3) durch berührungsloses Dispensen und insbesondere durch Tintenstrahldruck aufgebracht wird.

9. Verfahren nach einem der vorhergehenden Ansprüche,
wobei mindestens eines der Kontaktpads (22-27) aus jedem Satz (200) ein Heizerkontaktpad (23) ist, das mit dem Heizer (34) verbunden ist,
- Anlegen eines Heizstroms an das Heizerkontaktpad (23), während der entsprechende Leistungsregler gesteuert wird, wodurch der Heizer (34) geheizt wird.

10. Verfahren nach einem der vorhergehenden Ansprüche,
wobei im Gassensor ein einmalig programmierbarer On-Chip-Speicher zum Speichern einer Heizroutine angeordnet ist, und
wobei als Reaktion auf das Zuführen des Stroms zu der Verarbeitungsschaltung (36) die entsprechende Leistungssteuerung gemäß der Heizroutine gesteuert wird.

11. Verfahren nach Anspruch 10, umfassend
- Anzeigen eines Zustands des Gassensorchips (3) durch ein Flag im einmalig programmierbaren On-Chip-Speicher,
- Steuerung des Leistungsstellers abhängig vom Flag, und
- insbesondere Steuern des Leistungsreglers nur dann, wenn das Flag einen nicht ausgeheilten Zustand des empfindlichen Materials anzeigt.

12. Gassensoranordnung umfassend
- mehrere Verarbeitungsschaltungen (36) und
- mehrere Gassensorchips (3), jeder der Gassensorchips (3), umfassend:
- einen Heizer (34),
- eine in oder mit dem Gassensorchip (3)integrierte Verarbeitungsschaltung (36),
- ein empfindliches Material, das auf den Sensorchip Gas aufgebracht wird
zum Aufbau einer für ein Gas empfindlichen Schicht (31) eine in den Gassensorchip (3) integrierte Leistungssteuerung, die von der Verarbeitungsschaltung (36) gesteuert wird,
wobei für jeden Gassensorchip (3) die entsprechende Verarbeitungsschaltung (36) in den Gassensorchip (3) integriert ist oder die Verarbeitungsschaltung (36) zusammen mit dem Gassensorchip (3) auf einem Substrat angeordnet ist, die Anordnung ferner umfassend
- einen Träger (2), wobei der Träger (2) ein gemeinsamer Träger (2) ist, auf dem mehrere Gassensorchips (3) montiert sind, wobei der gemeinsame Träger (2) Sätze von Kontaktpads (200) aufweist, wobei jeder Satz von Kontaktpads (200) einen der mehrere Gassensorchips (3) elektrisch kontaktiert, wobei eines der Kontaktpads (25) aus dem Satz (200) ein Versorgungskontaktpad (25) ist, um der Verarbeitungsschaltung (36) des entsprechenden Gassensorchips elektrischen Strom zuzuführen,
- elektrische Zwischenverbindungen (251) zum elektrischen Verbinden eines Satzes von Versorgungskontaktpads (250), um die Zufuhr eines Stroms zu allen Versorgungskontaktpads (25) des Satzes (250) und somit zu den entsprechenden Verarbeitungsschaltungen (36) zu ermöglichen.

13. Gassensoranordnung nach Anspruch 12,
wobei der Satz von Versorgungskontaktpads (250) benachbarten Gassensorchips (3) entspricht,
wobei die elektrischen Zwischenverbinder (251) vorzugsweise Drahtverbindungen sind.

## Revendications

1. Procédé de fabrication d'un capteur de gaz,
comprenant les étapes de
- intégrer un circuit de traitement (36) dans un puce de capteur de gaz (3) ou agencement d'un circuit de traitement (36) avec une puce de capteur de gaz (3) sur un substrat (37),
- intégrer un réchauffeur (34) dans le capteur de gaz puce (3),
- appliquer d'un matériau sensible au puce de capteur de gaz, pour constituer une couche sensible au gaz,
- monter de plusieurs puces de capteur de gaz (3) sur un support (2), lequel support (2) est un support commun (2) configuré pour accepter les multiples puces de capteur de gaz (3), dans lequel le support commun (2) comprend des ensembles (200) de plots de contact (22-27), dans lequel chaque ensemble de plots de contact (200) contacte électriquement l'un des plusieurs puces de capteur de gaz (3), dans lequel l'une des plots de contact (22-27) de chaque ensemble (200) est un plot de contact d'alimentation (25) pour fournir du courant électrique au circuit de traitement (36) de la puce de capteur de gaz correspondante (3),
- interconnecter électriquement un ensemble de plots de contact d'alimentation (250) par des interconnecteurs électriques (251),
- appliquer un courant à au moins l'un des plots de contact d'alimentation (25) hors de l'ensemble (250) et via le interconnecteurs (251) à toutes les plots de contact d'alimentation (25) de l'ensemble (250), pour alimenter un courant aux circuits de traitement (36) correspondants,
- en réponse à l'application du courant, contrôler un régulateur de puissance, qui est intégré dans la puce de capteur de gaz correspondante, par le circuit de traitement (36), et
- en réponse au contrôle du régulateur, permettre d'alimenter un courant au réchauffeur (34), pour recuire la couche sensible correspondante (31).

2. Procédé selon la revendication 1,
dans lequel l'ensemble de plots de contact d'alimentation (250) comprend des plots de contact d'alimentation (25) de puces de capteur de gaz voisines (3).

3. Procédé selon l'une quelconque des revendications précédentes, dans lequel l'ensemble des plots de contact d'alimentation (250) correspond à des puces de capteur de gaz (3) qui sont alignées à la suite,
de préférence dans lequel les interconnexions électriques (251) sont fabriquées par connexions par fils.

4. Procédé selon l'une quelconque des revendications précédentes, comprenant
- appliquer pour chaque puce de capteur de gaz (3) un composé de moulage (1) enveloppant au moins partiellement la puce de capteur de gaz (3) générant ainsi une ouverture (11) permettant d'accéder à une partie de la puce de capteur de gaz (3) étant découverte par le composé de moulage (1),
- appliquer le matériau sensible pour chaque puce de capteur de gaz (3) après l'application du composé de moulage (1),
- appliquer le matériau sensible à travers l'ouverture (11) sur la partie découverte de la puce de capteur de gaz (3).

5. Procédé selon l'une quelconque des revendications précédentes,
dans lequel le support (2) comprend des pastilles de connexion (21) pour monter les puces de capteur de gaz (3) sur un premier sous-ensemble de fils de support (41, 411) connectés aux plots de contact (22-27) et un deuxième sous-ensemble de fils de support des conducteurs (42, 421) connectés aux pastilles de connexion (21),
le procédé comprenant
- une première étape de sectionnement, sectionnant le premier sous-ensemble de conducteurs de support (41, 411) avant de recuire la couche sensible (31),
- une deuxième étape de sectionnement, sectionnant les interconnexions électriques (251) et le deuxième sous-ensemble de conducteurs de support (42, 421), après recuit des couches sensibles (31), pour séparer les puces de capteur de gaz (3),
en particulier dans lequel la première et/ou la deuxième étape de séparation est réalisée par sciage.

6. Procédé selon la revendication 5,
dans lequel la première étape de séparation est appliquée après l'application du matériau sensible et/ou après l'application du composé de moulage (1).

7. Procédé selon la revendication 5,
dans lequel les puces de capteur de gaz (3) sont testées et/ou calibrées après avoir recuit les couches sensibles (31) et avant la deuxième étape de sectionnement.

8. Procédé selon la revendication 4,
dans lequel le matériau sensible est appliqué sur la partie découverte de la puce de capteur de gaz (3) par distribution sans contact, et en particulier par impression à jet d'encre.

9. Procédé selon l'une quelconque des revendications précédentes,
dans lequel au moins l'un des plots de contact (22-27) de chaque ensemble (200) est un plot de contact chauffant (23), connecté au réchauffeur (34),
- appliquer un courant de chauffage au plot de contact de chauffage (23) tandis que le régulateur de puissance correspondant est commandé, chauffant ainsi le réchauffeur (34).

10. Procédé selon l'une quelconque des revendications précédentes,
dans lequel une mémoire sur puce programmable une seule fois est agencée dans le capteur de gaz, pour stocker une routine de chauffage, et
dans lequel en réponse à la fourniture du courant au circuit de traitement (36), le régulateur de puissance correspondant est commandé selon la routine de chauffage.

11. Procédé selon la revendication 10, comprenant
- indiquer un état de la puce de détection de gaz (3) par un indicateur dans la mémoire sur puce programmable une seule fois,
- contrôler le régulateur de puissance en fonction d'indicateur, et
- en particulier, commander le régulateur de puissance uniquement lorsque l'indicateur indique un état non recuit du matériau sensible.

12. Réseau de capteurs de gaz comprenant
- plusieurs circuits de traitement (36), et
- plusieurs puces de capteur de gaz (3), chacune des puces de capteur de gaz (3) comprenant:
- un réchauffeur (34),
- un circuit de traitement (36) intégré dans ou avec la puce de capteur de gaz (3),
- un matériau sensible, appliqué sur la puce de capteur de gaz (3) pour constituer une couche (31) sensible à un gaz,
- un régulateur de puissance, intégré dans la puce du capteur de gaz (3), qui est commandé par le circuit de traitement (36),
dans lequel pour chaque puce de capteur de gaz (3), le circuit de traitement correspondant (36) est intégré dans la puce de capteur de gaz (3) ou le circuit de traitement (36) est agencé avec la puce de capteur de gaz (3) sur un substrat,
le réseau comprenant en outre
- un support (2), lequel support (2) est un support commun (2) sur lequel plusieurs puces de capteur de gaz (3) sont montées,
dans lequel le support commun (2) définit des plots de contact (200), dans lequel chaque ensemble des plots de contact (200) contactent électriquement l'une des multiples puces de capteur de gaz (3), dans lequel l'un des plots de contact (25) de l'ensemble (200) est un plot de contact d'alimentation (25), pour fournir un courant électrique au circuit de traitement (36) de la puce du capteur de gaz correspondant,
- des interconnecteurs électriques (251) pour interconnecter électriquement un ensemble de plots de contact d'alimentation (250), pour permettre l'alimentation en courant de toutes les plages de contact d'alimentation (25) de l'ensemble (250) et donc des circuits de traitement (36) correspondants.

13. Réseau de capteurs de gaz selon la revendication 12,
dans lequel l'ensemble de plots de contact d'alimentation (250) correspond à des puces de détection de gaz voisines (3),
dans lequel les interconnexions électriques (251) sont de préférence des connexions par fils.
